# EUROPEAN PATENT SPECIFICATION

(11) **EP 2 391 353 B1**
(45) Date of publication and mention of the grant of the patent: **10.02.2016**
(21) Application number: 09787579.3
(22) Date of filing: 16.03.2009
(51) Int. Cl.: A61K 9/28, A61K 31/495

(54) **PHARMACEUTICAL COMPOSITIONS OF TRIMETAZIDINE**
PHARMAZEUTISCHE ZUSAMMENSETZUNGEN VON TRIMETAZIDIN
COMPOSITIONS PHARMACEUTIQUES DE TRIMÉTAZIDINE

(30) Priority: 30.01.2009 IN KO01752009
(43) Date of publication of application: 07.12.2011
(73) Proprietor: Lupin Limited, Maharashtra (IN)
(72) Inventor: BHUTADA, Pravin, Meghrajji, Maharashtra (IN); DESHMUKH, Ashish, Ashokrao, Maharashtra (IN); DALAL, Satish , Kumar, Maharashtra (IN); KULKARNI, Shirishkumar, Maharashtra (IN)
(74) Representative: Adamson Jones
(86) International application number: PCT/IN2009/000180
(87) International publication number: WO 2010/086868

(56) References cited:
- EP-A- 0 673 649
- EP-A- 1 108 424
- EP-A- 1 195 160

## Description

### FIELD OF THE INVENTION

The present invention relates to once daily sustained release pharmaceutical compositions of Trimetazidine or a pharmaceutically acceptable salt(s) using dual retard release technique.

### BACKGROUND OF THE INVENTION

Trimetazidine is an antianginal drug and was first disclosed in US patent 3262852.

Trimetazidine dihydrochloride [1-(2,3,4-trimethoxybenzyl)-piperazine dihydrochloride] is freely soluble in water, about 80%. It has two pKa values 4.32 and 8.95. It regulates ionic and extra cellular exchanges, correcting the abnormal flow of ions across the cell membrane caused by ischemia and preventing cellular edema caused by anoxia. Thus it ensures the functioning of the ion pumps and the sodium-potassium transmembrane flux and maintains the cellular homeostasis.

Trimetazidine dihydrochloride is used therapeutically, as a coronary vasodilator for the prophylactic treatment of anginal chest pain attack and during such attacks, during chorioretinal attacks as well as for the treatment of giddiness of vascular origin (Vertigo of Maniere, acouphenous).

Trimetazidine dihydrochloride is administered orally in doses of 60 to 70 mg daily in divided doses as an immediate or modified release preparation. It is quickly absorbed and eliminated by the organism with plasma half-life of around 6.0 ± 1.4 hours and Tₘₐₓ of around 1.8 ± 0.7 hours. Since it has a shorter plasma half life, in practice 20mg preparation is given twice or thrice a day in order to ensure relatively constant plasma levels but, due to the fact that it is absorbed quickly, these immediate release forms lead to maximum plasma levels immediately after administration and to a very low plasma level at the time of the next dose, resulting in great differences in peak and trough plasma levels at steady state. Trimetazidine dihydrochloride is regarded as a safe drug in the long treatment of chronic ischemic disorders. This compels the necessity of fabricating the immediate release dosage form into a sustained release once-a-day preparation for achieving regular and constant plasma levels, which is also favourable for compliance of the patient to his treatment.

Trimetazidine is currently marketed as modified release tablets at the dosage of 35 mg under the brand name "Vastarel^{®}".

U.S. Patent 4,814,176 by Makino, Yuji; Matugi, Hideo; Suzuki, Yoshiki; describes a sustained release preparation comprising a) chitin, chitosan or a mixture thereof or b) non-anionic cellulose ether, and c) pharmaceutically effective amount of at least one pharmaceutically active agent.

U.S. Patent 4,755,544 by Makino, Yuji; Matugi, Hideo; Suzuki, Yoshiki; describes a sustained release preparation comprising (a) at least one non-anionic cellulose ether, (b) at least one anionic polymer compound selected from the group consisting of methoxyethylene-maleic anhydride copolymers and the hydrolyzates thereof, and (c) at least one pharmaceutically active agent..

European Patent Application 0673649 by Huet de Barochez describes pharmaceutical compositions for the prolonged release of trimetazidine or of one of its pharmaceutically acceptable salts characterized in that prolonged release of trimetazidine is controlled by the use of a mixture of water insoluble polymer and a plasticizer coated on a reservoir system containing 80mg of trimetazidine dihydrochloride.

European Patent Application 1108424 B1 by Servier Lab describes matrix tablet for the prolonged release of trimetazidine or a pharmaceutically acceptable salt thereof, characterized in that the prolonged release is controlled by the use of a cellulose derivative polymer present in the matrix, selected from hydroxypropylcellulose, hydroxyethylcellulose, hydroxymethylcellulose, methylcellulose and hydroxypropyl methylcellulose.

European Patent Application 1195160 by USV describes a sustained release composition of trimetazidine dihydrochloride comprising at least one of one or more hydrocolloid forming materials, one or more hydrophobic polymers and one or more other categories of hydrophobic materials.

However, there are alternate means by which we can design a once daily pharmaceutical composition of trimetazidine which is bioequivalent to twice daily compositions marketed under the brand name, Vastarel^{®} 35mg.

Thus, the present invention provides a once daily sustained release formulation of trimetazidine or its pharmaceutically acceptable salts thereof using dual retard technique, which provide freedom from burst effect, associated with formulation of controlled release of highly water-soluble drugs. Further the present invention reduces the fluctuation in peak-valley plasma concentration-time profile compared to marketed formulation.

### OBJECTS OF THE INVENTION

The object of the present invention is a once daily dual retard sustained release pharmaceutical composition comprising a matrix core comprising trimetazidine or a pharmaceutically acceptable salt(s), one or more sustained release polymer(s) and one or more pharmaceutically acceptable excipient(s), wherein the core is further coated with an extended release coating.

Another object of the present invention is a once daily dual retard sustained release pharmaceutical composition comprising a matrix core comprising trimetazidine or a pharmaceutically acceptable salt(s), one or more sustained release polymer(s) and one or more pharmaceutically acceptable excipient(s), wherein the core is further coated with an extended release coating, wherein about 0-55% of drug released in about 4 hours, about 30-85% of drug released in about 8 hours, and greater than about 75% of drug released in about 12 hours using 0.1 N HCl as dissolution medium.

Another object of the present invention is a once daily dual retard sustained release pharmaceutical composition comprising a matrix core comprising trimetazidine or a pharmaceutically acceptable salt(s), one or more sustained release polymer(s) and one or more pharmaceutically acceptable excipient(s), wherein the core is further coated with an extended release coating characterized in that the once daily sustained release pharmaceutical composition is bioequivalent to 35mg twice daily modified release formulation.

Another object of the present invention is a once daily dual retard sustained release pharmaceutical composition comprising a matrix core comprising trimetazidine or a pharmaceutically acceptable salt(s), one or more sustained release polymer(s) and one or more pharmaceutically acceptable excipient(s), wherein the core is further coated with an extended release coating ,wherein the fluctuation in peak-valley plasma concentration-time profile is reduced compared to 35mg twice daily modified release formulation.

Another object of the present invention is a once daily dual retard sustained release pharmaceutical composition comprising a matrix core comprising trimetazidine or a pharmaceutically acceptable salt(s), one or more sustained release polymer(s) and one or more pharmaceutically acceptable excipient(s) wherein the core is further coated with an extended release coating said pharmaceutical composition providing an in vivo plasma profile selected from (a) Mean Cmax is 50 -170 ng/ml, (b) Mean Tmax 5.5 - 15 hours (c) Mean AUC (0-48 hrs) is 1000 - 2400ng/ml and (d) Mean AUC (0- ∞) is 1000 - 2400ng/ml.

Another object of the present invention is a once daily dual retard sustained release pharmaceutical composition comprising a matrix core comprising trimetazidine or a pharmaceutically acceptable salt(s), one or more sustained release polymer(s) and one or more pharmaceutically acceptable excipient(s) wherein the core is further coated with an extended release coating, wherein the dosage form is compressed using a punch size of at least 12mm.

### DETAILED DESCRIPTION OF THE INVENTION

The present invention relates to a once daily dual retard sustained release pharmaceutical composition comprising a matrix core comprising trimetazidine or a pharmaceutically acceptable salt(s), one or more sustained release polymer(s) and one or more pharmaceutically acceptable excipient(s), wherein the core is further coated with an extended release coating.

As is well known, the maximum time effectiveness in many pharmaceutical preparations containing a drug is only a few hours because of biological modification and elimination of the medication in the body. Consequently, repeated doses must be taken at frequent intervals to obtain long term therapeutic levels of drugs. After high initial peak concentrations, the level of drug in the blood stream continually decreases due to biological elimination, so there is little or no therapeutic effect at the end of the period between doses. As a result, the therapeutic effect fluctuates between doses corresponding to the peaks and valleys in the level of drug in blood.

The problem with the marketed formulation is that blood levels of trimetazidine over the entire 24 hour dosing period are not maintained. Blood levels of trimetazidine fall significantly before the next dose is administered resulting in a significant variance between peak and valley levels. Accordingly formulation of, our invention affords a reduction in peak-valley fluctuation and in the number of daily intakes from twice daily to once daily.

As used herein, "pharmaceutically acceptable salts" refer to derivatives of the trimetazidine wherein trimetazidine is modified by reacting it with an acid or base as needed to form an ionically bound pair. Examples of pharmaceutically acceptable salts include conventional non- toxic salts or the quaternary ammonium salt of the parent compound formed, for example, from non-toxic inorganic or organic acids. Suitable non-toxic salts include those derived from inorganic acids such as hydrochloric, hydrobromic, sulfuric, sulfamic, phosphoric, nitric, and others known to those of ordinary skill in the art. The salts prepared from organic acids such as amino acids, acetic, propionic, succinic, glycolic, stearic, lactic, malic, tartaric, citric, ascorbic, pamoic, maleic, hydroxymaleic, phenylacetic, benzoic, salicylic, sulfanilic, fumaric, oxalic, isethionic, and others known to those of ordinarily skilled in the art. List of other suitable salts are found in Remington's Pharmaceutical Sciences, 17th edition. Mack Publishing Company, Easton Pa., 1985. p. 1418, the relevant disclosure of which is hereby incorporated by reference. The most preferred salt of Trimetazidine for the present invention is dihydrochloride salt.

The term "sustained release pharmaceutical compositions " as used herein in relation to the composition according to the invention means release, which is not immediate release and is taken to encompass controlled release, sustained release, prolonged release, timed release, retarded release, extended release and delayed release. The term " sustained release pharmaceutical compositions" as used herein can be described as pharmaceutical compositions whose drug-release characteristics of time course and/or location are chosen to accomplish therapeutic or convenience objectives not offered by conventional dosage forms such as a solution or an immediate release dosage form. Modified release solid oral dosage forms include both delayed and extended release drug products (as per US FDA guideline for 'SUPAC-MR: Modified Release Solid Oral Dosage Forms'). Sustained release can be used interchangeably with prolonged release, programmed release, timed release, extended release, controlled release and other such dosage forms.

By "pharmaceutically acceptable" is meant a carrier comprised of a material that is not biologically or otherwise undesirable.

Presently, trimetazidine is administered 35 milligrams orally two times a day as modified release dosage form. The therapeutic dose according to the present invention for once daily formulation can vary from 50-100 mg per day, preferably 60-80mg per day and most preferably 70mg once daily.

"Cₘₐₓ" as used herein, means maximum plasma concentration of the trimetazidine.

"Tₘₐₓ" as used herein, means time taken to achieve the maximum observed plasma concentration.

"AUC" as used herein, means area under the plasma concentration-time curve.

The sustained release polymers in the core comprise one or more hydrophilic or hydrophobic polymer(s) or combinations thereof.

The hydrophilic polymers include but are not limited to hydroxyethylcellulose, hydroxypropyl cellulose, Hydroxypropyl Methylcellulose, sodium carboxymethyl cellulose, sodium alginate, carbomer (Carbopol(TM)), xanthan gum, guar gum, locust bean gum, poly vinyl acetate, polyvinyl alcohol. Swellable polymers include, but are not limited to, a crosslinked poly (acrylic acid), crosslinked poly (alkylene oxide), crosslinked polyvinyl alcohol, a crosslinked polyvinyl pyrrolidone; a polyurethane hydrogel, a maleic anhydride polymer, such as a maleic anhydride copolymer, a cellulose polymer, a polysaccharide, starch, and starch based polymers, alginates, alginic acid, PVA, carbomer. The most preferred hydrophilic polymer in the core according to the present invention is xanthan gum.

The hydrophobic polymers include but are not limited to hydrogenated vegetable oil, purified grades of beeswax; fatty acids; long chain fatty alcohols, such as cetyl alcohol, myristyl alcohol, and stearyl alcohol; glycerides such as glyceryl esters of fatty acids like glyceryl monostearate, glyceryl distearate, glyceryl esters of hydrogenated castor oil and the like; oils such as mineral oil and the like, or acetylated glycerides; ethyl cellulose, stearic acid , paraffin, carnauba wax, talc; and the stearate salts such as calcium, magnesium, zinc and other materials known to one of ordinary skill in the art.

Pharmaceutically acceptable excipients include but are not limited to binders, diluents, lubricants, disintegrants, glidants and surface-active agents.

The amount of excipient employed will depend upon how much active agent is to be used. One excipient can perform more than one function.

Binders include, but are not limited to, starches such as potato starch, wheat starch, corn starch; microcrystalline cellulose such as products known under the registered trade marks Avicel, Filtrak, Heweten or Pharmacel; celluloses such as hydroxypropyl cellulose, hydroxyethyl cellulose, hydroxypropylmethyl cellulose (HPMC), ethyl cellulose, sodium carboxy methyl cellulose; natural gums like acacia, alginic acid, guar gum; liquid glucose, dextrin, povidone, syrup, polyethylene oxide, polyvinyl pyrrolidone, poly-N-vinyl amide, polyethylene glycol, gelatin, poly propylene glycol, tragacanth, combinations there of and other materials known to one of ordinary skill in the art and mixtures thereof.

Fillers or diluents, which include, but are not limited to confectioner's sugar, compressible sugar, dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic, calcium sulphate, and the like can be used.

Lubricants may be selected from, but are not limited to, those conventionally known in the art such as Mg, Al or Ca or Zn stearate, polyethylene glycol, glyceryl behenate, mineral oil, sodium stearyl fumarate, stearic acid, hydrogenated vegetable oil and talc.

Glidants include, but are not limited to, silicon dioxide; magnesium trisilicate, powdered cellulose, starch, talc and tribasic calcium phosphate, calcium silicate, magnesium silicate, colloidal silicon dioxide, silicon hydrogel and other materials known to one of ordinary skill in the art.

Disintegrants include but not limited to starches; clays; celluloses; alginates; gums; cross-linked polymers, e.g., cross-linked polyvinyl pyrrolidone or crospovidone, e.g., POLYPLASDONE XL, cross-linked sodium carboxymethylcellulose or croscarmellose sodium, e.g., AC-DI-SOL from FMC; and cross-linked calcium carboxymethylcellulose; soy polysaccharides; and guar gum. Use of disintegrant according to the present invention facilitates in the release of drug in the latter stage and thereby completely releasing the drug from the dosage form.

The present compositions may optionally contain a surface-active agent. The preferred agent is copolymers composed of a central hydrophobic chain of polyoxypropylene (poly (propylene oxide)) and polyoxyethylene (poly (ethylene oxide)) that is well kown as poloxamer. However, other agents may also be employed such as dioctyl sodium sulfosuccinate (DSS), triethanolamine, sodium lauryl sulphate (SLS), polyoxyethylene sorbitan and poloxalkol derivatives, quaternary ammonium salts or other pharmaceutically acceptable surface-active agents known to one ordinary skilled in the art.

The pharmaceutical dosage forms of the invention have an extended release coating, This coating helps pharmaceutical formulations to release the drug at and for the required time.

The extended release coating comprises a hydrophilic or hydrophobic substance(s) or the combinations thereof. Most preferably, a combination of hydrophilic and hydrophobic substance(s) is used.

It has been found that a simple matrix composition of trimetazidine dihydrochloride using one or more sustained release polymer(s) cannot sustain the release of the drug over the desired time period, however it was found that a combination of sustained release matrix core and an extended release coating helps in release of the drug for the entire duration of release required.

The pharmaceutical composition according to the present invention therefore uses the dual release retard technique. The dual retard technique is a combination of matrix and reservoir formulations. First the matrix particles of active ingredient and one or more sustained release polymer(s) are formed and then, these are further coated with an extended release coating. Thus, the dual retard release technique presents the double barriers and effectively controls the diffusion of the high dose, high solubility active ingredients from the present invention in predictable manner.

The other advantages of the present invention are such as it reduces the chances of dose dumping, unnecessary burst effects and failure of the system, which are otherwise usually associated with simple matrix or reservoir systems.

The hydrophobic substance in the coating is selected from but are not limited to Ammonio methacrylate copolymers type A and B as described in USP, methacrylic acid copolymer type A, B and C as described in USP, Polyacrylate dispersion 30% as described in Ph. Eur., Polyvinyl acetate dispersion, ethylcellulose, cellulose acetate, cellulose propionate (lower, medium or higher molecular weight), cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), and poly(hexyl methacrylate). Poly(isodecyl methacrylate), poly(lauryl methacrylate), poly(phenyl methacrylate), poly(methyl acrylate), poly(isopropyl acrylate), poly(isobutyl actylate), poly(octadecyl acrylate), waxes such as beeswax, carnauba wax, microcrystalline wax, and ozokerite; fatty alcohols such as cetostearyl alcohol, stearyl alcohol; cetyl alcohol and myristyl alcohol; and fatty acid esters such as glyceryl monostearate, glycerol distearate; glycerol monooleate, acetylated monoglycerides, tristearin, tripalmitin, cetyl esters wax, glyceryl palmitostearate, glyceryl behenate, and hydrogenated castor oil.

The hydrophilic substance in the coating is selected from but are not limited to celluloses or their salts or derivatives thereof, hydroxyethylcellulose, hydroxypropyl cellulose, hydroxypropyl methylcellulose, sodium carboxymethyl cellulose, alginic acid or their salts and derivatives thereof, carbomer (Carbopol(TM)), polyethyleneoxide, xanthan gum, guar gum, locust bean gum, poly vinyl acetate, polyvinyl alcohol, lactose, PVA these hydrophilic polymers also act as pore forming agent.

According to a most preferred embodiment the extended release coating contains a combination of ethylcellulose and hydroxypropylmethylcellulose.

Pharmaceutical dosage forms of the invention can be coated by a wide variety of methods. Suitable methods include compression coating, coating in a fluidized bed or a pan and hot melt (extrusion) coating. Such methods are well known to those skilled in the art.

These coating comprises one or more excipients selected from the group comprising coating agents, opacifiers, fillers, plasticizers, polishing agents, colouring agents, antitacking agents and the like.

The pharmaceutical formulation according to the present invention include but is not limited to tablets (single layered tablets, multilayered tablets, mini tablets, bioadhesive tablets, caplets, matrix tablets, tablet within a tablet, mucoadhesive tablets, modified release tablets, pulsatile release tablets, timed release tablets), pellets, beads, granules, sustained release formulations, capsules, microcapsules, tablets in capsules and microspheres, matrix formulations, microencapsulation and powder/pellets/granules for suspension.

The pharmaceutical composition of the invention can be formed by various methods known in the art such as by dry granulation, wet granulation, melt granulation, direct compression, double compression, extrusion spheronization, layering and the like.

The solvent(s) used in wet granulation in the present invention include all the solvents well known in the art or their mixtures thereof.

Trimetazidine is basically absorbed from the upper part of the stomach which is its primary site of absorption. Therefore the size of the punch which is used for compression also plays a major role in the pharmaceutical composition of the present invention. It is most preferred to use a bigger punch size for the tablets of the present invention and thereby providing the retention of the drug in the upper part of the stomach.

The gastric sphincter has a size of 12mm so any punch size which is 12mm or above it is used for the present invention. Most preferably the punch used in the present invention is 13.2mm.

Sustained release matrix tablet according to the present invention are manufactured preferably as per the following procedure:
i) blend the trimetazidine and pharmaceutically acceptable additives,
ii) subjecting the blend to slugging/compaction to form a coprimate
iii) converting the coprimate to granules and
iv) Mixing the coprimate with other excipients
v) Compressing the granules to form the solid oral dosage form
vi) The compressed granules are further coated with an extended release coating.

The in-vivo study of the trimetazidine formulation of the present invention containing a dosage of 70 mg trimetazidine revealed the following pharmacokinetics parameters wherein the mean peak plasma concentration (Cmax) of drug in the blood remains from about 50 to about 170 ng/ml, mean Tmax from about 5.5 to about 15 hours, mean AUC (0-48hrs) from about 1000 to about 2400ng/ml and mean AUC (0-∞) from about 1000 to about 2400ng/ml. The examples given below are illustrative embodiments of the invention and are merely exemplary. A person skilled in the art may make variations and modifications without deviating from scope of the invention. All such modifications and variations are intended to be included within the scope of the invention.

### EXAMPLES

### Example 1:

| **S.No** | **Ingredient** | **Qty (mg/tab)** |
|---|---|---|
| 1 | Trimetazidine DiHCL | 70 |
| 2 | Xanthan Gum | 500 |
| 3 | Lactose | 160 |
| 4 | Avicel | 160 |
| 5 | Povidone | 50 |
| 6 | Avicel | 40 |
| 7 | Aerosil | 10 |
| 8 | Magnesium Stearate | 10 |
| | Total | 1000 |
| | **Coating** | |
| 9 | Ethyl cellulose | 22.5 |
| 10 | HPMC | 22.5 |
| 11 | Triethyl citrate | 5 |
| 12 | Isopropyl alcohol | q.s |
| 13 | Dichloromethane | q.s |

### Procedure:

1) Sift the Lactose & Avicel along with Trimetazidine DiHCL.
2) Mix the materials of step 1 & granulate it with povidone water solution to produce granules.
3) Sift together Xanthan Gum & Avicel mix well with granules of step 2.
4) Sift the Magnesium Stearate & Aerosil.
5) Lubricate the blend of step 3 with sifted material of step 4
6) Compress the blend of step 5.

### Coating:

1) Take Sufficient qty of IPA & DCM
2) Add required quantity of HPMC, EC & Triethyl citrate, to above solvent under continuous stirring to dissolve.
3) Coat the core tablets.

### Example 2:

| **S.No** | **Ingredient** | **Qty (mg/tab)** |
|---|---|---|
| 1 | Trimetazidine DiHCL | 70 |
| 2 | Lactose (Intra) | 100 |
| 3 | Lactose (Extra) | 150 |
| 4 | Avicel | 460 |
| 5 | Hydrogenated Vegetable Oil | 200 |
| 6 | Aerosil | 10 |
| 7 | Magnesium Stearate | 10 |
| | **Total** | 1000 |
| 8 | Ethyl cellulose | 22.5 |
| 9 | HPMC | 22.5 |
| 10 | Triethyl citrate | 5 |
| 11 | Isopropyl alcohol | q.s |
| 12 | Dichloromethane | q.s |

### Procedure:

1. Melt Hydrogenated Vegetable Oil to the melted add Trimetazidine DiHCL & lactose (intra)
2. Allow the materials of the step 1 to cool & solidify, mill it get granules
3. Mix the granules of step 2 & with extra granular materials, Avicel & lactose
4. Sift the Magnesium Stearate & Aerosil.
5. Lubricate the blend of step 3 with sifted material of step 4
6. Compress the blend of step 5.

### Coating:

1. Take Sufficient qty of IPA & DCM
2. Add required quantity of HPMC, EC & Triethyl citrate, to above solvent under continuous stirring to dissolve.
3. Coat the core tablets

### Example 3:

| **S.No** | **Ingredient** | **Qty (mg/tab)** |
|---|---|---|
| 1 | Trimetazidine DiHCL | 70 |
| 2 | Xanthan Gum | 200 |
| 3 | Lactose (intra) | 160 |
| 4 | Avicel | 250 |
| 5 | Dicalcium Phosphate | 300 |
| 6 | Aerosil | 10 |
| 7 | Magnesium Stearate | 10 |
| | **Total** | 1000 |
| 8 | Ethyl cellulose | 22.5 |
| 9 | HPMC | 22.5 |
| 10 | Triethyl citrate | 5 |
| 11 | Isopropyl alcohol | q.s |
| 12 | Dichloromethane | q.s |

### Procedure:

1. Sift Trimetazidine dihydrochloride and sufficient quantity of lactose. To the above mixture, add Magnesium Stearate.
2. Compress the slug of above blend using suitable punch & suitable hardness.
3. Deslug the slug of step 3 and sift it.
4. Sift the DCP, Xanthan gum, lactose, Avicel, Aerosil together. And mix well with blend of step 4.
5. Sift the magnesium Stearate. And lubricate the blend of step. 5.
6. Compress the blend of step. 6 using suitable punch.

### Coating:

1. Take Sufficient qty of IPA & DCM
2. Add required quantity of HPMC, EC & Triethyl citrate, to above solvent under continuous stirring to dissolve.
3. Coat the core tablets.

### Example 4:

| **S.No** | **Ingredient** | **Qty (mg/tab)** |
|---|---|---|
| 1 | Trimetazidine DiHCL | 70 |
| 2 | Lactose | 400 |
| 3 | Magnesium Stearate | 5 |
| 4 | Xanthan gum | 70 |
| 5 | Croscarmellose sodium | 100 |
| 6 | Lactose | 230 |
| 7 | Microcrystalline cellulose | 110 |
| 8 | Colloidal silicon dioxide | 10 |
| 9 | Magnesium Stearate | 5 |
| | **Coating** | |
| 10 | Ethyl cellulose | 22.5 |
| 11 | HPMC | 22.5 |
| 12 | Triethyl citrate | 5 |
| 13 | Isopropyl alcohol | q.s |
| 14 | Dichloromethane | q.s |

### Procedure:

1. Sift Trimetazidine dihydrochloride and sufficient quantity of lactose through sieve.
2. To the above mixture add Magnesium Stearate.
3. Compress the slug of above blend using suitable punch & suitable hardness.
4. Deslug the slug of step 3 and sift it.
5. Sift the Croscarmellose Sodium, xanthan gum, lactose, Avicel, Aerosil together. And mix well with blend of step 4.
6. Sift the magnesium stearate. And lubricate the blend of step 5.
7. Compress the blend of step 6 using suitable punch.

### Coating:

1. Take Sufficient qty of IPA & DCM
2. Add required quantity of HPMC, EC & Triethyl citrate, to above solvent under continuous stirring to dissolve.
3. Coat the core tablets

### Example 5:

| **S.No** | **Ingredient** | **Qty (mg/tab)** |
|---|---|---|
| 1 | Trimetazidine DiHCL | 70 |
| 2 | Lactose | 400 |
| 3 | Magnesium Stearate | 5 |
| 4 | Xanthan gum | 70 |
| 5 | Croscarmellose sodium | 100 |
| 6 | Lactose | 230 |
| 7 | Microcrystalline cellulose | 110 |
| 8 | Colloidal silicon dioxide | 10 |
| 9 | Magnesium Stearate | 5 |
| | **Coating** | |
| 10 | Eudragit | 25 |
| 12 | Triethyl citrate | 5 |
| 13 | Isopropyl alcohol | q.s |
| 14 | Acetone | q.s |

### Procedure:

1. Sift Trimetazidine dihydrochloride and sufficient quantity of Lactose through sieve.
2. To the above mixture add Magnesium Stearate.
3. Compress the slug of above blend using suitable punch & suitable hardness.
4. Deslug the slug of step 3. and sift it.
5. Sift the Crosscarmellose Sodium, xanthan gum, lactose, microcrystalline cellulose, Colloidal silicon dioxide together. And mix well with blend of step 4.
6. Sift the magnesium stearate. And lubricate the blend of step 5.
7. Compress the blend of step 6 using suitable shape punch.

### Coating:

1. Take Sufficient qty of IPA & Acetone
2. Add required quantity of Eudragit & Triethyl citrate, to above solvent under continuous stirring to dissolve.
3. Coat the core tablets

### In-vitro Dissolution Study

The compositions of the present invention have a prolonged in vitro release rate compared to the marketed Vastarel® composition. The in vitro test used to measure release rate of the active agent from a composition of the invention is as follows:
A solution of 900 ml of a 0.1N HCl and the apparatus USP Dissolution Apparatus Type I. The tablet composition was placed in the apparatus and dissolution was periodically measured. The in vitro dissolution studies of Example 4 is such that about 30% to about 85 % of drug is released in about 8 hrs, and more than about 85% of drug is released in 12 hours.

| **Time (hr)** | **Cumulative % Drug Release** |
|---|---|
| 1 | About 1- About 7 |
| 2 | About 8- About 15 |
| 4 | About 33- About 44 |
| 6 | About 52- About 68 |
| 8 | About 70- About 85 |
| 10 | About 82- About 95 |
| 12 | About 85 - About 100 |

### In -vivo Bioequivalence Study

Open Label, balanced, randomized, single-dose, two-treatment, two-sequence, two-period crossover relative bioavilability study of Trimetazidine 70 mg MR Tablets example 4 (once daily) of Lupin Limited, India Comparing with that of Vastarel® (Trimetazidine) MR Tablets 35mg bid (one Tablets each 12 hourly) of Servier Industries, France, in 12 healthy, adult, male, human subjects under fasting conditions.

The study was designed to demonstrate the similar clinical efficacy compared to Vastarel^{®}.

The plasma Trimetazidine concentration is shown in **Figure: 1**

*The in-vivo* bioequivalence study shows the results as shown in the table below:

**Table 1: Comparative pharmacokinetic parameters of present invention vs Vastarel®**

| | **AUC (0-48hour) ng/ml** | **AUC (0-∞) ng/ml** | **Cₘₐₓ ng/ml** | **Tₘₐₓ (hrs)** |
|---|---|---|---|---|
| **Example 4** | 1607.95 | 1642.02 | 94.26 | 8.50 |
| **Vastarel 35 mg MR (bid)** | 1779.58 | 1812.34 | 87.84 | 10.00 |

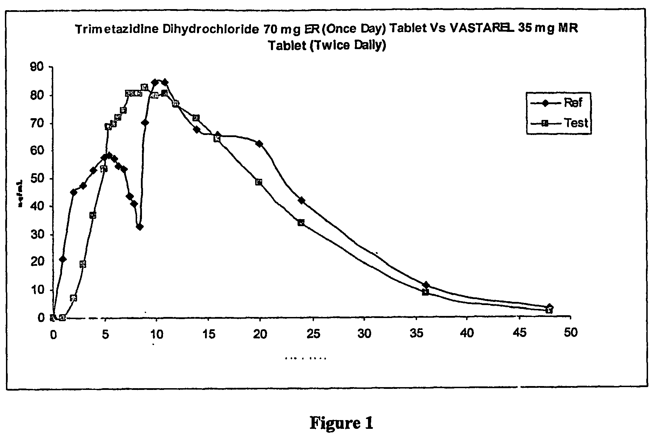

## Claims

1. A once daily dual retard sustained release pharmaceutical composition comprising a matrix core comprising trimetazidine or a pharmaceutically acceptable salt(s), one or more sustained release polymer(s) and one or more pharmaceutically acceptable excipient(s), wherein the core is further coated with an extended release coating.

2. The once daily dual retard sustained release pharmaceutical composition according to claim 1, wherein one or more sustained release polymer(s) comprises hydrophilic and/or hydrophobic polymer or combinations thereof.

3. The once daily dual retard sustained release pharmaceutical composition according to claim 1, wherein trimetazidine is present in a dose selected from a range of 50-100mg.

4. The once daily dual retard sustained release pharmaceutical composition according to claim 1, wherein trimetazidine is present in a dose selected from a range of 60-80mg.

5. The once daily dual retard sustained release pharmaceutical composition according to claim 1, wherein trimetazidine is present in a dose of 70mg.

6. The once daily dual retard sustained release pharmaceutical composition according to claim 1, wherein the extended release coating comprises hydrophilic and/or hydrophobic substances or combinations thereof.

7. The once daily dual retard sustained release pharmaceutical composition according to claim 6, wherein the hydrophilic substance is hydroxypropylmethylcellulose and the hydrophobic substance is ethyl cellulose.

8. The once daily dual retard sustained release pharmaceutical composition according to claim 1, wherein about 0-55% of drug released in about 4 hours, about 30-85% of drug released in about 8 hours, and greater than about 75% of drug released in about 12 hours using 0.1 N HCl as dissolution medium.

9. The once daily dual retard sustained release pharmaceutical composition according to claim 1, wherein the once daily sustained release pharmaceutical composition is bioequivalent to 35mg twice daily modified release formulation.

10. The once daily dual retard sustained release pharmaceutical composition according to claim 1, wherein the fluctuation in peak-valley plasma concentration-time profile is reduced compared to 35mg twice daily modified release formulation.

11. The once daily dual retard sustained release pharmaceutical composition according to claim 1, said pharmaceutical composition providing an in vivo plasma profile selected from (a) Mean Cmax from about 50 to about 170 ng/ml, (b) Mean Tmax from about 5.5 to about 15 hours (c) Mean AUC (0-48 hrs) from about 1000 to about 2400ng/ml and (d) Mean AUC (0- ∞) from about 1000 to about 2400ng/ml.

12. The once daily dual retard sustained release pharmaceutical composition according to claim 1, wherein the dosage form is compressed using a punch size of at least 12mm.

13. A process for preparing a once daily dual retard sustained release pharmaceutical composition according to claim 1, wherein the process comprises the steps of blending trimetazidine or a pharmaceutically acceptable salt(s) and pharmaceutically acceptable additives, subjecting the blend to slugging/granulation, mixing with other excipient(s) and then compressing the granules to form the solid oral dosage form using a punch size of at least 12mm which is then further coated with an extended release coating.

## Patentansprüche

1. Pharmazeutische Einmal-täglich-Dual-Retard-Zusammensetzung mit verzögerter Freisetzung, die einen Matrix-Kern beinhaltet, der Trimetazidin oder ein pharmazeutisch akzeptable(s) Salz(e) davon, ein oder mehrere Polymere mit verzögerter Freisetzung und einen oder mehrere pharmazeutisch akzeptable Hilfsstoffe beinhaltet, wobei der Kern weiter mit einer Beschichtung mit verlängerter Freisetzung beschichtet ist.

2. Pharmazeutische Einmal-täglich-Dual-Retard-Zusammensetzung mit verzögerter Freisetzung gemäß Anspruch 1, wobei ein oder mehrere Polymere mit verzögerter Freisetzung hydrophiles und/oder hydrophobes Polymer oder Kombinationen davon beinhalten.

3. Pharmazeutische Einmal-täglich-Dual-Retard-Zusammensetzung mit verzögerter Freisetzung gemäß Anspruch 1, wobei Trimetazidin in einer Dosis ausgewählt aus einem Bereich von 50-100 mg vorliegt.

4. Pharmazeutische Einmal-täglich-Dual-Retard-Zusammensetzung mit verzögerter Freisetzung gemäß Anspruch 1, wobei Trimetazidin in einer Dosis ausgewählt aus einem Bereich von 60-80 mg vorliegt.

5. Pharmazeutische Einmal-täglich-Dual-Retard-Zusammensetzung mit verzögerter Freisetzung gemäß Anspruch 1, wobei Trimetazidin in einer Dosis ausgewählt aus einem Bereich von 70 mg vorliegt.

6. Pharmazeutische Einmal-täglich-Dual-Retard-Zusammensetzung mit verzögerter Freisetzung gemäß Anspruch 1, wobei die Beschichtung mit verlängerter Freisetzung hydrophile und/oder hydrophobe Substanzen oder Kombinationen davon beinhaltet.

7. Pharmazeutische Einmal-täglich-Dual-Retard-Zusammensetzung mit verzögerter Freisetzung gemäß Anspruch 6, wobei die hydrophile Substanz Hydroxypropylmethylcellulose ist und die hydrophobe Substanz Ethylcellulose ist.

8. Pharmazeutische Einmal-täglich-Dual-Retard-Zusammensetzung mit verzögerter Freisetzung gemäß Anspruch 1, wobei etwa 0-55 % des Arzneimittels, freigesetzt in etwa 4 Stunden, etwa 30-85 % des Arzneimittels, freigesetzt in etwa 8 Stunden, mehr als 75 % des Arzneimittels, freigesetzt in etwa 12 Stunden, 0,1 N HCl als Auflösungsmedium verwenden.

9. Pharmazeutische Einmal-täglich-Dual-Retard-Zusammensetzung mit verzögerter Freisetzung gemäß Anspruch 1, wobei die Einmal-täglich-Zusammensetzung mit verzögerter Freisetzung bioäquivalent zu 35 mg-Zweimal-täglich-Formulierung mit modifizierter Freisetzung ist.

10. Pharmazeutische Einmal-täglich-Dual-Retard-Zusammensetzung mit verzögerter Freisetzung gemäß Anspruch 1, wobei die Fluktuation im Spitze-Tal-Plasma-Konzentration-Zeit-Profil verglichen mit der 35 mg-Zweimal-täglich-Formulierung mit modifizierter Freisetzung reduziert wird.

11. Pharmazeutische Einmal-täglich-Dual-Retard-Zusammensetzung mit verzögerter Freisetzung gemäß Anspruch 1, wobei die pharmazeutische Zusammensetzung ein In-vivo-Plasma-Profil bereitstellt, ausgewählt aus (a) mittlere Cmax von etwa 50 bis etwa 170 ng/ml, (b) mittlere Tmax von etwa 5,5 bis etwa 15 Stunden, (c) mittlere AUC (0-48 Std.) von etwa 1000 bis etwa 2400 ng/ml und (d) mittlere AUC (0-∞) von etwa 1000 bis etwa 2400 ng/ml.

12. Pharmazeutische Einmal-täglich-Dual-Retard-Zusammensetzung mit verzögerter Freisetzung gemäß Anspruch 1, wobei die Dosierungsform unter Verwendung einer Stempelgröße von mindestens 12 mm komprimiert ist.

13. Verfahren zum Herstellen einer Einmal-täglich-Dual-Retard-Zusammensetzung mit verzögerter Freisetzung gemäß Anspruch 1, wobei das Verfahren die folgenden Schritte beinhaltet: Vermengen von Trimetazidin oder eines pharmazeutisch akzeptablen Salzes bzw. akzeptabler Salze und eines pharmazeutisch akzeptablen Zusatzes bzw. akzeptabler Zusätze, Unterwerfen der Mischung einem Slugging/einer Granulierung, Mischen mit anderen Hilfsstoffen und dann Komprimieren der Granulate, um die feste orale Dosierungsform unter Verwendung einer Stempelgröße von mindestens 12 mm zu bilden, die dann weiter mit einer Beschichtung mit verlängerter Freisetzung beschichtet wird.

## Revendications

1. Composition pharmaceutique à libération prolongée à double retard à prise quotidienne unique comprenant un noyau de matrice qui comporte de la trimétazidine ou un ou plusieurs sels pharmaceutiquement acceptables, un ou plusieurs polymères pour libération prolongée et un ou plusieurs excipients pharmaceutiquement acceptables, ledit noyau étant enrobé d'un enrobage pour libération prolongée.

2. Composition pharmaceutique à libération prolongée à double retard à prise quotidienne unique selon la revendication 1, lesdits un ou plusieurs polymères pour libération prolongée comprenant un polymère hydrophile et/ou un polymère hydrophobe ou des combinaisons de ceux-ci.

3. Composition pharmaceutique à libération prolongée à double retard à prise quotidienne unique selon la revendication 1, ladite trimétazidine étant présente en une dose choisie dans une plage de 50 à 100 mg.

4. Composition pharmaceutique à libération prolongée à double retard à prise quotidienne unique selon la revendication 1, ladite trimétazidine étant présente en une dose choisie dans une plage de 60 à 80 mg.

5. Composition pharmaceutique à libération prolongée à double retard à prise quotidienne unique selon la revendication 1, ladite trimétazidine étant présente en une dose de 70 mg.

6. Composition pharmaceutique à libération prolongée à double retard à prise quotidienne unique selon la revendication 1, ledit enrobage pour libération prolongée comprenant des substances hydrophiles et/ou des substances hydrophobes ou des combinaisons de celles-ci.

7. Composition pharmaceutique à libération prolongée à double retard à prise quotidienne unique selon la revendication 6, ladite substance hydrophile étant l'hydroxypropylméthylcellulose et ladite substance hydrophobe étant l'éthylcellulose.

8. Composition pharmaceutique à libération prolongée à double retard à prise quotidienne unique selon la revendication 1, environ 0 à 55 % de médicament étant libérés en environ 4 heures, environ 30 à 85 % de médicament étant libérés en environ 8 heures et plus d'environ 75 % de médicament étant libérés en environ 12 heures en utilisant du HCl à 0,1 N comme milieu de dissolution.

9. Composition pharmaceutique à libération prolongée à double retard à prise quotidienne unique selon la revendication 1, ladite composition pharmaceutique à libération prolongée à prise quotidienne unique étant bioéquivalente à une formulation de 35 mg à libération modifiée à double prise quotidienne.

10. Composition pharmaceutique à libération prolongée à double retard à prise quotidienne unique selon la revendication 1, lesdites fluctuations du profil de concentration plasmatique pic-creux en fonction du temps étant réduites comparativement à la formulation de 35 mg à libération modifiée à double prise quotidienne.

11. Composition pharmaceutique à libération prolongée à double retard à prise quotidienne unique selon la revendication 1, ladite composition pharmaceutique fournissant un profil plasmatique in vivo choisi parmi (a) une Cmax moyenne d'environ 50 à environ 170 ng/ml, (b) une Tmax moyenne d'environ 5,5 à environ 15 heures, (c) une ASC moyenne (0 à 48 heures) d'environ 1000 à environ 2400 ng/ml et (d) une ASC moyenne (0 à ∞) d'environ 1000 à environ 2400 ng/ml.

12. Composition pharmaceutique à libération prolongée à double retard à prise quotidienne unique selon la revendication 1, ladite forme pharmaceutique étant comprimée en utilisant une taille de poinçon d'au moins 12 mm.

13. Procédé de préparation d'un composition pharmaceutique à libération prolongée à double retard à prise quotidienne unique selon la revendication 1, ledit procédé comprenant les étapes consistant à mélanger de la trimétazidine ou un ou plusieurs sels pharmaceutiquement acceptables et des additifs pharmaceutiquement acceptables, soumettre le mélange à un briquetage/une granulation, mélanger avec un ou plusieurs autres excipients et ensuite comprimer les granulés pour former une forme pharmaceutique solide orale en utilisant une taille de poinçon d'au moins 12 mm qui est ensuite enrobée avec un enrobage pour libération prolongée.
